# EUROPEAN PATENT APPLICATION

(11) **EP 4 801 232 A1**
(43) Date of publication of application: **02.09.2026**
(21) Application number: 24882479.9
(22) Date of filing: 25.10.2024
(51) Int. Cl.: H10K 30/50, C07D 487/22, H10K 30/40, H10K 30/81, H10K 30/86, H10K 50/14, H10K 85/10, H10K 85/30, H10K 85/50

(54) **PHOTOELECTRIC CONVERSION ELEMENT AND PHOTOELECTRIC CONVERSION DEVICE**

(30) Priority: 27.10.2023 JP 2023184761; 27.10.2023 JP 2023184756; 27.10.2023 JP 2023184750; 21.12.2023 JP 2023216294; 21.12.2023 JP 2023216296; 21.12.2023 JP 2023216299; 16.02.2024 JP 2024022244; 16.02.2024 JP 2024022251; 16.02.2024 JP 2024022246; 28.05.2024 JP 2024086004; 23.10.2024 JP 2024186452
(71) Applicant: Canon Kabushiki Kaisha, Tokyo 146-8501 (JP)
(72) Inventor: YOSHIDA, Yu, Tokyo 146-8501 (JP); MAKISUMI, Kohei, Tokyo 146-8501 (JP); KATO, Nanami, Tokyo 146-8501 (JP); SEKIDO, Kunihiko, Tokyo 146-8501 (JP); NISHIDA, Tsutomu, Tokyo 146-8501 (JP)
(74) Representative: TBK
(86) International application number: PCT/JP2024/038056
(87) International publication number: WO 2025/089371

(57) **Abstract**

Provided is a photoelectric conversion element having improved conversion efficiency and durability.

The photoelectric conversion element is a photoelectric conversion element including: a first electrode; a second electrode; and a photoelectric conversion layer arranged between the first electrode and the second electrode, the photoelectric conversion layer containing a crystal having a perovskite structure, wherein the photoelectric conversion element includes, between the photoelectric conversion layer and the first electrode, a layer containing a crystal of a cyclic conjugated compound in which pyrrole rings are bonded by conjugated bonds, wherein the crystal of the cyclic conjugated compound in which the pyrrole rings are bonded by conjugated bonds contains an organic compound, and wherein the organic compound has a pKa of 10.0 or more and a dipole moment of 1.60 D or more.

## Description

### [Technical Field]

The present invention relates to a photoelectric conversion element and a photoelectric conversion apparatus.

### [Background Art]

Recently, an n-p diode-type silicon (Si) single crystal-based solar cell has been widely used as a solar cell having high energy conversion efficiency in solar power generation. However, the solar cell requires a high temperature treatment step and the price of a material itself is high, and hence there is a problem in that the cost per unit electric power is high.

To solve those problems, a solar cell using an organic material has been intensively investigated. Of those, a perovskite solar cell using a crystal having a perovskite structure as a photoelectric conversion layer has attracted attention because of its high photoelectric conversion efficiency. The perovskite crystal to be used in the perovskite solar cell has a problem in that its stability against heat, humidity, long-term light irradiation, or the like is low, and hence an improvement in durability has been required. In Patent Literature 1, an investigation has been made to form a layer for protecting a perovskite layer by incorporating a highly insulating polymer into a hole-transporting layer.

However, as a result of investigations made by the inventors of the present invention, such highly insulating material still has a problem in stably achieving both photoelectric conversion efficiency and durability, such as a significant deterioration in photoelectric conversion when the thickness of a layer formed of the material is increased.

### [Citation List]

### [Patent Literature]

PTL 1: Japanese Patent No. 6943591

### [Summary of Invention]

### [Technical Problem]

As described above, the formation of a layer intended to protect a perovskite layer between the perovskite layer and an electrode has been used as means for solving the problem of durability. However, there is also a problem in that the addition of such layer hinders the movement of charge generated in the perovskite layer. The hindrance leads to a reduction in photoelectric conversion efficiency.

Accordingly, the present invention is directed to improving the durability and photoelectric conversion efficiency of each of a photoelectric conversion element and a photoelectric conversion apparatus by protecting a perovskite structure from light, heat, and moisture.

### [Solution to Problem]

The present invention is directed to a photoelectric conversion element including:
a first electrode;
a second electrode; and
a photoelectric conversion layer arranged between the first electrode and the second electrode, the photoelectric conversion layer containing a crystal having a perovskite structure,
wherein the photoelectric conversion element includes, between the photoelectric conversion layer and the first electrode, a layer containing a crystal of a cyclic conjugated compound in which pyrrole rings are bonded by conjugated bonds,
wherein the crystal of the cyclic conjugated compound in which the pyrrole rings are bonded by conjugated bonds contains an organic compound, and wherein the organic compound has a pKa of 10.0 or more and a dipole moment of 1.60 Debye (D) or more.

### [Advantageous Effects of Invention]

According to the present invention, the photoelectric conversion element having improved conversion efficiency and durability can be provided.

### [Brief Description of Drawings]

[Fig. 1]

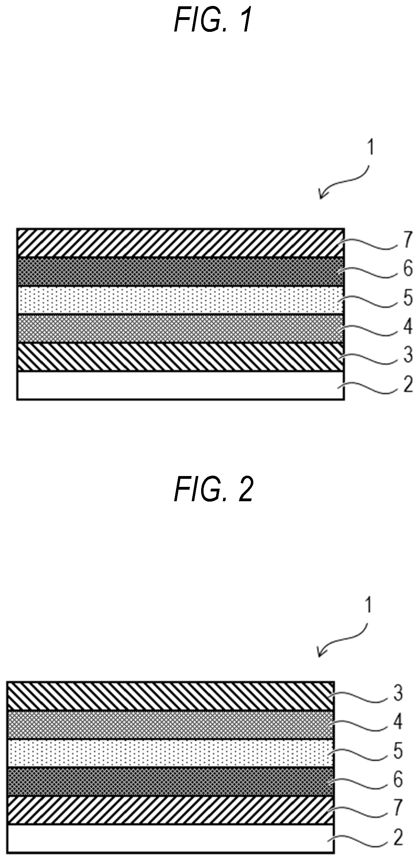

   Fig. 1 is a schematic sectional view in the thickness direction of a photoelectric conversion element according to a first embodiment of the present invention.
[Fig. 2]
   Fig. 2 is a schematic sectional view in the thickness direction of a photoelectric conversion element according to a second embodiment of the present invention.
[Fig. 3]

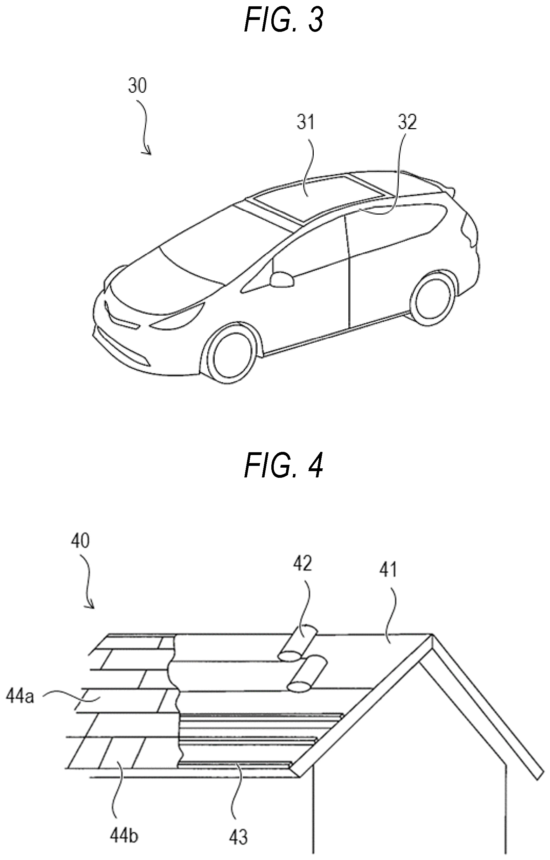

   Fig. 3 is a perspective view for schematically illustrating a moving body including the photoelectric conversion element according to one embodiment of the present invention.
[Fig. 4]
   Fig. 4 is a perspective view for schematically illustrating a building material including the photoelectric conversion element according to one embodiment of the present invention.

### [Description of Embodiments]

The present invention is described in detail below by way of preferred embodiments. The present invention is not limited to the following embodiments, and the following embodiments, which are appropriately changed, modified, and the like based on the ordinary knowledge of a person skilled in the art without departing from the gist of the present invention, are also encompassed within the scope of the present invention.

The term "layer" as used herein means not only a layer having a clear boundary or a layer having a flat thin film shape but also a layer having a concentration gradient in which the concentration of a constituent element gradually changes, or a layer that may form a complicatedly intricate structure together with another layer. In addition, the elemental analysis of the layer may be performed by, for example, performing the TOF-SIMS/FE-TEM/EDS line analysis measurement of a cross section of the photoelectric conversion element and determining the element distribution of a specific element.

The present invention is directed to a photoelectric conversion element including: a first electrode; a second electrode; and a photoelectric conversion layer arranged between the first electrode and the second electrode, the photoelectric conversion layer containing a crystal having a perovskite structure, the element being characterized in that: the photoelectric conversion element includes, between the photoelectric conversion layer and the first electrode, a layer containing a crystal of a cyclic conjugated compound in which pyrrole rings are bonded by conjugated bonds; the crystal of the cyclic conjugated compound in which the pyrrole rings are bonded by conjugated bonds contains an organic compound; and the organic compound has a pKa of 10.0 or more and a dipole moment of 1.60 Debye (D) or more.

The inventors of the present invention have found that the photoelectric conversion element has the above-mentioned characteristics and hence can have improved photoelectric conversion efficiency and durability. The inventors have conceived those effects to be as described below.

A compound having a crystal of a cyclic conjugated compound in which pyrrole rings are bonded by conjugated bonds has a high hole-transporting property. Accordingly, charge generated in a perovskite layer can be quickly transported by combining a layer containing such compound with the perovskite layer. When an organic compound having a pKa of 10.0 or more and a dipole moment of 1.60 Debye (D) or more is incorporated into the crystal, such effect is more significantly exhibited. In the case where the pKa is 10.0 or more, when an organic compound having a dipole moment of 1.60 D or more is present close to the cyclic conjugated compound, the organic compound is expected to interact with a π-electron of the cyclic conjugated compound to cause a charge bias. As a result, it is conceived that polarization is promoted, and hence the mobility of charge is improved. When the dipole moment of an organic compound is high, its molecule is easily polarized, and hence a charge bias is easily generated. Further, when the pKa is high, it is conceived that basicity is high, and hence the charge bias is more easily increased. The pKa of the organic compound in the crystal of the cyclic conjugated compound of the present invention is characterized by being 10.0 or more, and the pKa is preferably 10.0 to 40.0.

The dipole moment of the organic compound is characterized by being 1.60 D or more, preferably 2.20 D or more. When the dipole moment falls within those ranges, the above-mentioned effect is easily exhibited, and hence the photoelectric conversion efficiency and the durability are easily improved. The organic compound preferably has a molar volume of 20.0 to 85.0 cm³/mol from the viewpoint of ease of incorporation into the crystal. When the molar volume is 85.0 cm³/mol or less, the organic compound can be incorporated into the crystal without greatly disrupting the crystal structure, and hence the photoelectric conversion efficiency is easily improved.

Various organic compounds, such as an amide, an ester, a ketone, an alcohol, a glycerin, a sulfoxide, and an organic amine, may each be used as the organic compound. Of those, an amide and a sulfoxide are preferred, and any one selected from the group consisting of: N-methylformamide; N-ethylformamide; N-propylformamide; and dimethyl sulfoxide is more preferred. When the crystal contains such organic compound, the photoelectric conversion efficiency is easily improved. With respect to such organic compound, the content of the organic compound is preferably 0.50 to 2.00 mass% with respect to the cyclic conjugated compound in which the pyrrole rings are bonded by conjugated bonds. When the content is 0.50 mass% or more, the above-mentioned effect is easily exhibited, and hence the photoelectric conversion efficiency is easily improved. In addition, when the content is 2.00 mass% or less, crystallinity is easily maintained, and hence the photoelectric conversion efficiency and the durability are easily improved. A method of introducing the organic compound into the crystal is not particularly limited, but is, for example, a method including causing the organic compound to coexist in a process of crystallization. A method of changing the amount of the organic compound in the crystal is, for example, a method including changing various conditions, such as a treatment time and a treatment intensity, in a crystallization process (e.g., Step 3 to be described later). For example, the amount of the organic compound can be reduced by increasing the treatment time after appropriately setting the treatment intensity.

The organic compound of the present invention is incorporated into the crystal. As a method of identifying and quantifying the organic compound incorporated into the crystal, verification may be performed by the following method.

The cyclic conjugated compound of the present invention is dispersed in a deuterated solvent in which the compound is insoluble, and is subjected to ¹H-NMR measurement, and the organic compound of the present invention is quantified. Next, the cyclic conjugated compound of the present invention is dissolved in a deuterated solvent in which the compound is soluble, and the kind and amount of the organic compound present in the crystal may be identified and quantified by taking a difference from the quantified value.

The cyclic conjugated compound in which the pyrrole rings are bonded by covalent bonds to be used in the present invention is preferably a porphyrin compound or a phthalocyanine compound, more preferably a phthalocyanine compound from the viewpoint of the spread of a π-electron cloud serving as the starting point of an interaction. The phthalocyanine compound preferably contains a central element, and examples of the central element include Ga, Cu, Ti, Zn, Si, V, Pb, and Pt. Of those, Ga, Ti, and Zn are preferred, and Ga is more preferred from the viewpoint of hole-transporting performance. The cyclic conjugated compound in which the pyrrole rings are bonded by covalent bonds may have an axial ligand as a ligand. OH, Cl, and O are each preferred as the axial ligand from the viewpoints of the spread of a π-electron cloud and the ease of taking a crystal. Further, a hydroxygallium phthalocyanine compound is more preferred as a combination of those central metals and axial ligands.

Specific examples of the cyclic conjugated compound in which the pyrrole rings are bonded by covalent bonds of the present invention include the following compounds.

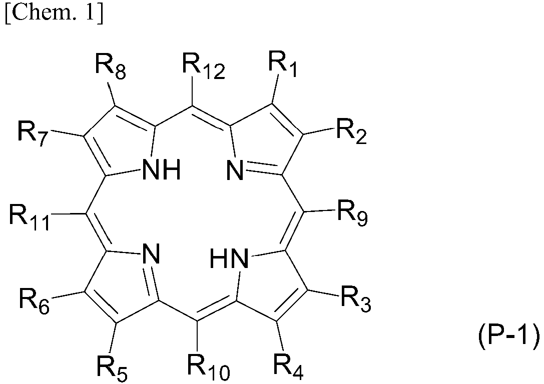

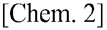

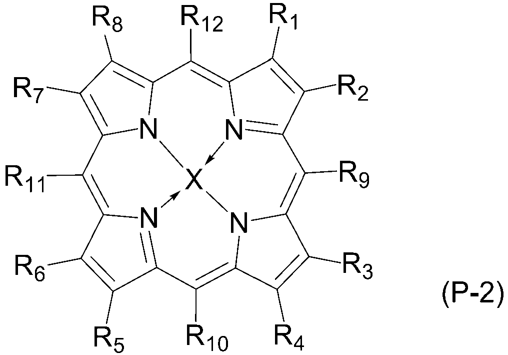

In the formulae (P-1) and (P-2), R₁ to R₁₂ each independently represent hydrogen, or an organic group including an aromatic group that may have a substituent or an aliphatic group that may have a substituent.

R₁ to R₁₂ each preferably represent hydrogen, a methyl group, an ethyl group, a propyl group, an isopropyl group, a butyl group, an octyloxy group, a butoxy group, a halogen atom, a phenyl group, a phenoxy group, a carboxyphenyl group, a benzenesulfonic acid group, a hydroxyphenyl group, a dihydroxyphenyl group, a trihydroxyphenyl group, a methoxyphenyl group, a dimethoxyphenyl group, a trimethoxyphenyl group, a methylphenyl group, a dimethylphenyl group, a trimethylphenyl group, a pyridyl group, an aminophenyl group, a sodium sulfonate group, a 4-cumylphenoxy group, a sulfonic acid group, a phenylthio group, a tert-butyl group, a hydroxy group, a carbonyl group, a methoxy group, an amino group, a sulfo group, or an aldehyde group. In the formulae (P-1) and (P-2), X represents a metal atom or an inorganic compound, and specifically, SiCl₂, Cu, Zn, Pd, Pb, Ni, Pt, Co, MnCl, FeCl, VO, and RuCO are preferred.

Specific examples of the phthalocyanine compound of the present invention include the following compounds.

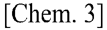

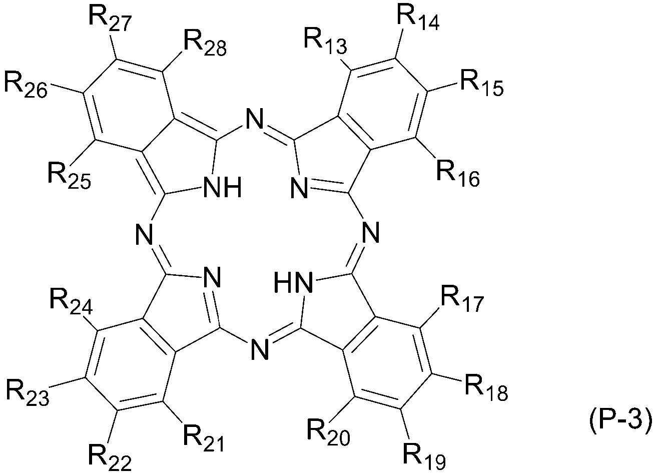

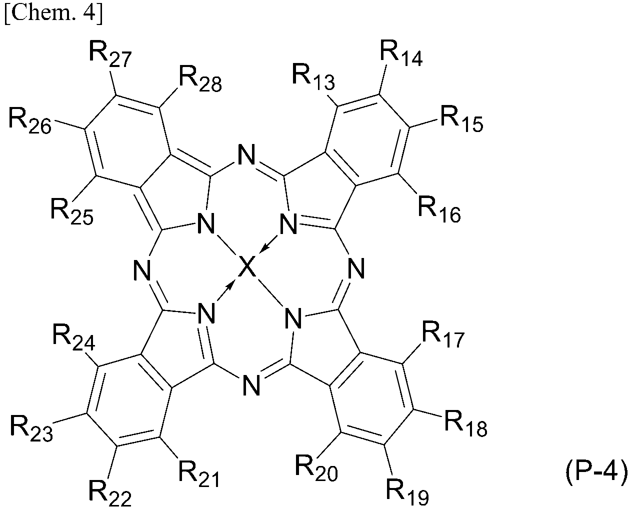

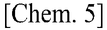

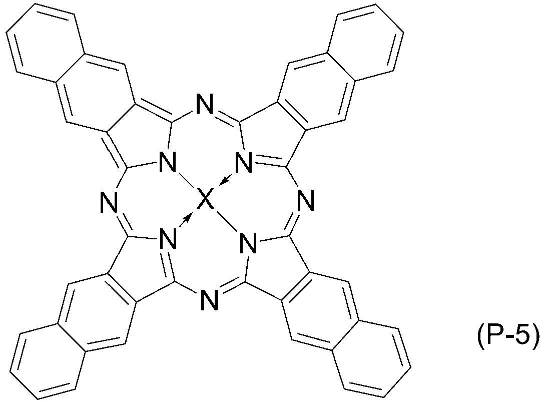

In the formulae (P-3) and (P-4), R₁₃ to R₂₈ each independently represent a hydrogen atom, or an organic group including an aromatic group that may have a substituent or an aliphatic group that may have a substituent. R₁₃ to R₂₈ each preferably represent a hydrogen atom, a methyl group, an ethyl group, a propyl group, an isopropyl group, a butyl group, an octyloxy group, a butoxy group, a halogen atom, a phenyl group, a phenoxy group, a carboxyphenyl group, a benzenesulfonic acid group, a hydroxyphenyl group, a dihydroxyphenyl group, a trihydroxyphenyl group, a methoxyphenyl group, a dimethoxyphenyl group, a trimethoxyphenyl group, a methylphenyl group, a dimethylphenyl group, a trimethylphenyl group, a pyridyl group, an aminophenyl group, a sodium sulfonate group, a 4-cumylphenoxy group, a sulfonic acid group, a phenylthio group, a tert-butyl group, a hydroxy group, a carbonyl group, a methoxy group, an amino group, a sulfo group, or an aldehyde group. Of those, a methyl group, an ethyl group, a propyl group, a butyl group, a halogen atom, a sulfonic acid group, a hydroxy group, a carbonyl group, a methoxy group, an amino group, a sulfo group, and an aldehyde group are preferred. In the formulae (P-3), (P-4), and (P-5), X represents a metal atom or an inorganic compound, and specifically, SiCl₂, Cu, Zn, Pd, Pb, Ni, Pt, Co, MnCl, FeCl, VO, and RuCO are preferred.

It has been found that a layer containing a crystal of a cyclic conjugated compound in which pyrrole rings are bonded by covalent bonds according to the present invention contributes to improvements in leak resistance and conversion efficiency even when a hole-transporting layer or an insulating layer having a thickness of several tens of nanometers is introduced between itself and a photoelectric conversion layer. The layer containing the crystal of the cyclic conjugated compound in which the pyrrole rings are bonded by covalent bonds according to the present invention is particularly preferably brought into contact with the photoelectric conversion layer as a charge-transporting layer or as part of the charge-transporting layer from the viewpoint of leak resistance. A compound to be used for the hole-transporting layer or the insulating layer that may be interposed between the layer containing the crystal of the cyclic conjugated compound in which the pyrrole rings are bonded by covalent bonds of the present invention and the photoelectric conversion layer is specifically preferably, for example, sodium chloride, sodium iodide, potassium iodide, rubidium iodide, cesium acetate, copper(I) bromide, copper(I) iodide, nickel(II) chloride, zinc iodide, germanium dioxide, aluminum acetylacetonate, europium(III) acetylacetonate, 1,8-diaminooctane dihydroiodide, 1,4-butanediamine dihydroiodide, hexylamine hydrobromide, n-octylamine hydrobromide, 2-phenylethylammonium iodide, ethylenediamine dihydroiodide, sodium fluoride, cesium chloride, methylammonium chloride, lead(II) thiocyanate, lead(II) acetate, potassium chloride, niobium(V) fluoride, choline chloride, L-α-phosphatidylcholine, fullerene, phenyl C61 butyric acid methyl ester (PCBM ((6,6)-phenyl C61 butyric acid methyl)), iodopentafluorobenzene, F4TCNQ, thiophene, pyridine, pentafluorobenzyl bromide, (3-mercaptopropyl)trimethoxysilane, thiourea, benzylamine, hexamethylenetetramine, N-(3-aminopropyl)-2-pyrrolidinone, theophylline, caffeine, 2-aminoethanesulfonamide hydrochloride, tri-n-octylphosphine oxide, graphene oxide, poly(3-hexylthiophene-2,5-diyl), poly(4-vinylpyridine), polyethylene oxide, polyvinylpyrrolidone, or poly(methyl methacrylate). Of those, sodium chloride, potassium iodide, rubidium iodide, cesium acetate, nickel(II) chloride, aluminum acetylacetonate, n-octylamine hydrobromide, 2-phenylethylammonium iodide, sodium fluoride, cesium chloride, methylammonium chloride, potassium chloride, niobium(V) fluoride, thiophene, pyridine, trimethoxysilane, thiourea, benzylamine, theophylline, poly(4-vinylpyridine), and poly(methyl methacrylate) are particularly preferred.

In addition, in the case where the layer having the cyclic conjugated compound in which the pyrrole rings are bonded by conjugated bonds contains a resin, when the content of the resin in the layer is represented by B and the content of the cyclic conjugated compound in which the pyrrole rings are bonded by conjugated bonds therein is represented by P, the ratio P/B is preferably 1 to 20.

Fig. 1 is a sectional view for schematically illustrating the configuration of the photoelectric conversion element according to one embodiment of the present invention. A case in which the layer containing the cyclic conjugated compound in which the pyrrole rings are bonded by conjugated bonds according to the present invention is a charge-transporting layer is described below. A photoelectric conversion element 1 includes a substrate 2, and a second electrode 3, an electron-transporting layer 4, a photoelectric conversion layer 5, a charge-transporting layer 6, and a first electrode 7 arranged thereon. One of the first electrode 7 and the second electrode 3 is a positive electrode, and the other is a negative electrode. A current can be extracted by connecting the first electrode 7 and the second electrode 3 to an external circuit.

The photoelectric conversion layer 5 is excited by light that has entered the layer through the substrate 2, the second electrode 3, and the electron-transporting layer 4, or the first electrode 7 and the charge-transporting layer 6 to generate an electron or a hole. That is, the photoelectric conversion layer 5 generates a current between the first electrode 7 and the second electrode 3. The electron-transporting layer 4 is a layer arranged between the photoelectric conversion layer 5, and the two electrodes 3 and 7, and may not be formed in some cases. A form in which the electron-transporting layers 4 and photoelectric conversion layers 5 are laminated may be adopted. Such form may also be referred to as "tandem structure." In addition, as illustrated in Fig. 2, the photoelectric conversion element may be produced in the order of the first electrode 7, the charge-transporting layer 6, the photoelectric conversion layer 5, the electron-transporting layer 4, and the second electrode 3 on the substrate 2.

The respective members for forming the photoelectric conversion element of the present invention are described below.

### [Photoelectric Conversion Element]

The photoelectric conversion element of the present invention is a photoelectric conversion element including: a first electrode; a second electrode; and a photoelectric conversion layer arranged between the first electrode and the second electrode, the photoelectric conversion layer containing a crystal having a perovskite structure, the photoelectric conversion element being characterized by including a charge-transporting layer between the photoelectric conversion layer and the first electrode. In addition, to improve the photoelectric conversion efficiency, a tandem type in which the photoelectric conversion elements are laminated may be adopted. The kind of the photoelectric conversion element to be laminated is not limited, and for example, a silicon solar cell or a CIGS solar cell may be adopted in addition to a perovskite solar cell using a crystal having a perovskite structure in its photoelectric conversion layer.

A method of forming each of the layers including the photoelectric conversion layer and charge-transporting layer of the photoelectric conversion element of the present invention is, for example, a coating method or a vapor deposition method. Examples of the coating method include dip coating, spin coating, spray coating, ink jet coating, meniscus coating, screen coating, roll coating, die coating, blade coating, curtain coating, and wire bar coating. The coating method is a method including preparing a coating liquid for each layer to be described later, applying the liquid in the desired order of layers, and drying the liquid. A desired method may be selected as such forming method in accordance with the respective layers.

The respective layers are described below.

### [Substrate]

The photoelectric conversion element 1 of the present invention may include the substrate 2, and examples thereof include a transparent glass substrate made of soda-lime glass or alkali-free glass, a ceramic substrate, and a transparent plastic substrate. When light is taken in from the first electrode 7 side, an opaque material may be used as the substrate 2, and when light is taken in from the second electrode 3 side, the substrate 2 is formed of a transparent material.

### [Electrode]

A material for the first electrode 7 or the second electrode 3 is not particularly limited, and a material that has hitherto been known may be used. Examples thereof include: metals, such as gold, silver, titanium, and copper; sodium; a sodium-potassium alloy; lithium; magnesium; carbon; a carbon nanotube; aluminum; a magnesium-silver mixture; a magnesium-indium mixture; an aluminum-lithium alloy; an Al/Al₂O₃ mixture; and an Al/LiF mixture. Examples of a transparent electrode material include: conductive transparent materials, such as CuI, indium tin oxide (ITO), SnO₂, aluminum zinc oxide (AZO), indium zinc oxide (IZO), gallium zinc oxide (GZO), fluorine-doped tin oxide (FTO), and antimony-doped tin oxide (ATO); and conductive transparent polymers. Those materials may be used alone or in combination thereof. At least one electrode of the first electrode 7 or the second electrode 3 on a light incident side is a transparent electrode, and the other may be a transparent electrode or may also serve as a reflective layer formed of a light reflective material, or may be a transparent electrode including a reflective layer on a side opposite to the light incident side. When the first electrode 7 is on the light incident side, the second electrode 3 and the substrate 2 may be a transparent electrode and a reflective layer, respectively. The transparent electrode may be a patterned electrode.

### [Photoelectric Conversion Layer]

The photoelectric conversion layer 5 contains the crystal having a perovskite structure. The crystal having a perovskite structure to be used in the present invention is preferably represented by the following general formula [1].

ABX₃ [1]

In the general formula [1], A represents a monovalent cation of an organic molecule or a metal atom, B represents a divalent metal cation, and X represents a monovalent halide anion.

A in the general formula [1] preferably represents CₚN_{q}Hᵣ ("p", "q", and "r" each represent a positive integer) in the case of, for example, the organic molecule. Specific examples thereof include methylammonium and formamidinium.

In addition, the metal atom is not particularly limited, and lithium, cesium, sodium, potassium, and rubidium are preferred. Those organic molecules or metal atoms may be used alone or in combination thereof.

When the cation A to be included is too large to fit in a crystal having a three-dimensional perovskite structure, a crystal having a two-dimensional perovskite structure, a crystal having a 2.5-dimensional perovskite structure with properties of both the two-dimensional and three-dimensional perovskite structures, a two-layer crystal having three-dimensional and two-dimensional perovskite structures, or a crystal having a mixed three-dimensional/two-dimensional perovskite structure is formed, and any of the structures functions as the photoelectric conversion layer. The two-layer crystal having three-dimensional and two-dimensional perovskite structures refers to a crystal in which the crystals having three-dimensional and two-dimensional perovskite structures are laminated as independent and separate layers. The crystal having a mixed three-dimensional/two-dimensional perovskite structure refers to a crystal having a structure in which both the regions or domains of crystals having two-dimensional or 2.5-dimensional layered and three-dimensional perovskite structures are mixed.

It is preferred that the crystal having a two-dimensional perovskite or 2.5-dimensional perovskite structure be represented by each of the following general formulae [2] to [4] ("n" represents a positive integer).

R'₂Aₙ₋₁BₙX₃ₙ₊₁ [2]

R"Aₙ₋₁BₙX₃ₙ₊₁ [3]

R‴₂AₙBₙX₃ₙ₊₁ [4]

The general formula [2], the general formula [3], and the general formula [4] form perovskite structures of a Ruddlesden-Popper (RP) type, a Dion-Jacobson (DJ) type, and an Alternating cations in the interlayer (ACI) type, respectively.

R', R", and R‴ in the general formulae [2] to [4] each represent a cation of an organic molecule or a metal that may have a substituent. Specifically, ethylammonium, propylammonium, n-butylammonium, n-hexylammonium, n-octylammonium, 1,6-hexanediammonium, iso-butylammonium, 3-(nonafluoro-tert-butyloxy)propylamine, 1,3-propanediammonium, 1,5-pentamethylenediamine, octyldiammonium, 2,2-(ethylenedioxy)bis(ethylammonium), 5-aminovaleric acid, 4-tert-butylammonium, N,N'-dimethylethylene-1,2-diammonium, 2,2,3,3,3-pentafluoropropylammonium, guanidinium, propylammonium, propargylamine, an alkylammonium, cyclohexylmethylammonium, 4-(aminomethyl)piperidinium, piperidinium, pyrrolidinium, cyclohexylammonium, 4-fluorophenethylammonium, 4-fluorophenethylammonium, trifluoromethylbenzylammonium, pentafluorobenzylammonium, pentafluorophenylethylammonium, 4-methoxyphenethylammonium, imidazolium, pyridinium, 3-thiophenemethylammonium, 2-thiopheneethylammonium, 2-thiopheneformamidinium, 2-thiophenemethylammonium, 1-naphthylmethylammonium, 2-naphthylmethylammonium, phenethylammonium, phenylammonium, benzylammonium, 2,5-thiophenedimethylammonium, phenylpropylammonium, 1,4-phenylenedimethanamine, 3-phenyl-2-propen-1-ammonium, phenylbutylammonium, 4-tert-butylbenzylammonium, 3-(aminomethyl)piperidinium, and 4-(aminomethyl)piperidinium are preferred.

B in each of the general formulae [1] to [4] represents a metal atom, and examples thereof include lead, tin, bismuth, zinc, titanium, antimony, nickel, iron, cobalt, silver, copper, gallium, germanium, magnesium, calcium, indium, aluminum, manganese, chromium, molybdenum, and europium. Of those, lead, tin, and bismuth are preferred from the viewpoint of the overlap of electron orbits. Those metal atoms may be used alone or in combination thereof.

X in each of the general formulae [1] to [4] represents a halogen atom, and examples thereof include chlorine, bromine, and iodine. Those halogen atoms may be used alone or in combination thereof. Of those, a halogen atom is preferred because, when the halogen atom is incorporated into the structure, the above-mentioned crystal having a perovskite structure easily becomes soluble in an organic solvent, and hence the application to an inexpensive printing method or the like is enabled. Further, iodine is more preferred because the energy bandgap of the crystal having a perovskite structure narrows.

Specifically, as three-dimensional perovskite, two-dimensional perovskite, and mixed three-dimensional/two-dimensional perovskite, MAPbI₃, FAPbCl₃, FAPbI₃, MAPbIₓBr₃₋ₓ, MAPbIₓCl₃₋ₓ, Cs_{0.05}(MA_{0.17}FA_{0.83})_{0.95}Pb(I_{0.83}Br_{0.17})₃, {Csₓ₁(FAₓ₂MA₁₋ₓ₂)₁₋ₓ₁}ₓ₃Pb(Iₓ₄Br₁₋ₓ₄)ₓ₅, Cs_{0.05}FA_{0.88}MA_{0.07}PbI_{2.56}Br_{0.44}, (FAPbI₃)_{0.95}(MAPbBr₃)_{0.05}, (FAPbI₃)_{0.85}(MAPbBr₃)_{0.15}, CsPbI₃, CsPbBr₃, Csₓ(MA)₁₋ₓPbI₃, Csₓ(FA)₁₋ₓPbI₃, MAₓ(FA)₁₋ₓPbI₃, MA_{0.17}FA_{0.83}Pb(I_{0.83}Br_{0.17})₃, Cs0.15FA0.85PbI2.55Br0.45, Cs0.05FA0.88MA0.07PbI2.56Br0.44, Cs0.15FA0.85PbI2.55Br0.45, (PEA)₂(MA)₂Pb₃I₁₀, (PTA)₂(MA)₄Pb₅I₁₆, (PEA)₂(MA)₄Pb₅I₁₆, (ThMA)₂(MA)₂Pb₃I₁₀, (3BBA)₂(MA)₂Pb₃I₁₀, (ThMA)₂(FA)₄Pb₅I₁₆, (4FPEA)₂(FA_{0.3}MA_{0.7})₄Pb₅I₁₆, (PDMA)FA₂Pb₃I₁₀, (3AMPY)(MA)₃Pb₄I₁₃, (PDMA)MA₅Pb₆I₁₉, (PDMA)MA₃Pb₄I₁₃, (TTDMA)MA₃Pb₄I₁₃, (TTDMA)MA₄Pb₅I_{16,} (BA_{0.9}PEA_{0.1})₂MA₄Pb₅I₁₆, (BA_{0.9}PEA_{0.1})₂MA₃Pb₄I₁₃, (4FPEA)₂MA₃Pb₄I₁₃, (4FPEA)₂MA₄Pb₅I₁₆, (BA)₂MA₂Pb₃I₁₀, (BA)₂MA₃Pb₄I₁₃, (TEA)₂MA₂Pb₃I₁₀, (BA)₂MA₄Pb₅I₁₆, (BA)₂MA₃Pb₄I₁₃, CsSnBr₃, CsSnI₃, FA_{0.75}MA_{0.25}Sn_{0.95}Ge_{0.05}I₃, FAMASnGeI₃, FASnBr₃, FASnI₃, MA₂Sn₃I₈, MASnBr₃, MASnGeI₃, and MASnI₃ are preferred. The A site, B site, or X site of each of the general formulae may be adjusted to be deficient or excessive in accordance with purposes, and the combinations of x1 to x5 may be changed in accordance with purposes. The combinations of x1 to x5 are, for example, as shown in Table 1. Particularly preferred ranges are 0.03≤x1≤0.10, 0.80≤x2≤0.96, 0.95≤x3≤1.05, 0.80≤x4≤0.96, and 2.95≤x5≤3.05. MACl may be included as a material for forming a perovskite crystal.

### [Table 1]

**Table 1**

| x1 | x2 | 1-x2 | x3 | x4 | 1-x4 | x5 |
|---|---|---|---|---|---|---|
| 0.05 | 0.83 | 0.17 | 1.00 | 0.83 | 0.17 | 3.00 |
| 0.05 | 0.83 | 0.17 | 0.99 | 0.83 | 0.17 | 2.99 |
| 0.05 | 0.83 | 0.17 | 0.98 | 0.83 | 0.17 | 2.98 |
| 0.05 | 0.83 | 0.17 | 0.97 | 0.83 | 0.17 | 2.97 |
| 0.05 | 0.83 | 0.17 | 0.96 | 0.83 | 0.17 | 2.96 |
| 0.05 | 0.83 | 0.17 | 1.01 | 0.83 | 0.17 | 3.01 |
| 0.05 | 0.83 | 0.17 | 1.02 | 0.83 | 0.17 | 3.02 |
| 0.05 | 0.83 | 0.17 | 1.03 | 0.83 | 0.17 | 3.03 |
| 0.05 | 0.83 | 0.17 | 1.04 | 0.83 | 0.17 | 3.04 |
| 0.05 | 0.83 | 0.17 | 1.00 | 0.95 | 0.05 | 3.00 |
| 0.05 | 0.83 | 0.17 | 0.97 | 0.95 | 0.05 | 2.97 |
| 0.05 | 0.83 | 0.17 | 0.98 | 0.95 | 0.05 | 2.98 |
| 0.05 | 0.83 | 0.17 | 0.99 | 0.95 | 0.05 | 2.99 |
| 0.05 | 0.83 | 0.17 | 1.01 | 0.95 | 0.05 | 3.01 |
| 0.05 | 0.83 | 0.17 | 1.02 | 0.95 | 0.05 | 3.02 |
| 0.05 | 0.83 | 0.17 | 1.03 | 0.95 | 0.05 | 3.03 |

In the above-mentioned specific examples, "MA" represents methylammonium, "FA" represents formamidinium, "PEA" represents phenethylammonium, "PTA" represents phenyltriethylammonium, "ThMA" represents 2-thiophenemethylammonium, "3BBA" represents 3-bromobenzylammonium, "3AMPY" represents 3-(aminomethyl)pyridine, "PDMA" represents 1,4-phenylenedimethanammonium, "TTDMA" represents thieno[3,2-b]thiophene-2,5-diyldimethanammonium, "4FPEA" represents 4-fluorophenethylammonium, "BA" represents butylammonium, and "TEA" represents 2-thiopheneethylammonium.

The above-mentioned crystal having a perovskite structure preferably has a cubic structure in which the metal atom B, the organic molecules A, and the halogen atom X are arranged on a body-centered position, the respective corners, and a face-centered position, respectively. The details are not clear, but it is assumed that, when such structure is present, the orientation of an octahedron in a crystal lattice can be easily changed, and hence the mobility of an electron in the crystal having a perovskite structure increases, and the photoelectric conversion efficiency of the photoelectric conversion element is improved.

The crystal having a perovskite structure to be used in the present invention is preferably a crystalline semiconductor. The term "crystalline semiconductor" means a semiconductor that enables the measurement of an X-ray scattering intensity distribution to detect a scattering peak. When the crystal having a perovskite structure is the crystalline semiconductor, the mobility of the electron in the crystal having a perovskite structure increases, and the photoelectric conversion efficiency of the photoelectric conversion element is improved.

The thickness of the photoelectric conversion layer according to the present invention is preferably 5 to 2,000 nm. When the thickness is 5 nm or more, light can be sufficiently absorbed, and when the thickness is 2,000 nm or less, the generated charge can be transported to the respective electrodes. A more preferred lower limit is 50 nm, a more preferred upper limit is 1,200 nm, a still more preferred lower limit is 100 nm, and a still more preferred upper limit is 1,000 nm.

### [Charge-transporting Layer]

The present invention is directed to a photoelectric conversion element including a charge-transporting layer, in which the layer containing the crystal of the cyclic conjugated compound in which the pyrrole rings are bonded by conjugated bonds according to the present invention is the charge-transporting layer. In this case, the thickness of the charge-transporting layer is preferably 1 to 1,000 nm, more preferably 5 to 500 nm, particularly preferably 10 to 200 nm.

As described above, when the charge-transporting layer contains a resin therein, the photoelectric conversion efficiency and the durability are easily improved. In the present invention, the term "resin" refers to a molecule having a number-average molecular weight (Mn) of 1,000 or more. The number-average molecular weight of the resin is preferably 10,000 or more.

The resin preferably has a glass transition temperature (Tg), and the Tg of the resin is preferably 95°C or less.

Examples of the resin to be preferably used in the present invention include a polyester-based resin, a polycarbonate-based resin, a polyvinyl acetal-based resin, a polyvinyl butyral-based resin, an acrylic resin, a polyvinyl alcohol-based resin, a cellulose-based resin, a polystyrene-based resin, a polyvinyl acetate-based resin, and a polyvinyl chloride-based resin. The content of the resin is preferably 1.0 to 20 mass% with respect to the cyclic conjugated compound.

The charge-transporting layer may be formed by preparing a coating liquid for a charge-transporting layer containing the above-mentioned respective materials and a solvent, forming a coating film of the coating liquid on the photoelectric conversion layer, and drying the coating film. Examples of the solvent to be used for the coating liquid include an alcohol-based solvent, a ketone-based solvent, an ether-based solvent, an ester-based solvent, and an aromatic hydrocarbon-based solvent. Of those solvents, an alcohol-based solvent or an aromatic hydrocarbon-based solvent is preferred.

### [Second Charge-transporting Layer]

In the present invention, the photoelectric conversion element 1 may further include the second charge-transporting layer between the charge-transporting layer 6 and the first electrode 7 from the viewpoint of the compatibility of a film of the charge-transporting layer 6.

A material for the second charge-transporting layer is not particularly limited, and examples thereof include a spirofluorene compound, a triphenylamine compound, a chrysene compound, a pyrene compound, a phthalocyanine compound, a carbazole compound, a fluorene compound, a phenylcyclohexane compound, a benzidine compound, a phenoxazine compound, a phenylenediamine compound, a thiocyanate compound, and a thiophene compound. The compound preferably has an aromatic ring from the viewpoint of the compatibility of a film interface, and Spiro-OMeTAD, PTAA, or a phthalocyanine compound is particularly preferred.

In addition, the second charge-transporting layer may contain a dopant as an additive in order to improve its charge transportation capability. Examples of a substance that may be used as the dopant include lithium compounds such as lithium bis(trifluoromethanesulfonyl)imide, cobalt compounds such as [tris(2-(1H-pyrazol-1-yl)-4-tert-butylpyridine)cobalt(III) tris(bis(trifluoromethylsulfonyl)imide)], boron compounds such as tetrakis(pentafluorophenyl)borate, molybdenum compounds such as tris[1-(methoxycarbonyl)-2-(trifluoromethyl)-ethane-1,2-dithiolene]molybdenum, organic compounds each having a tetracyanoquinodimethane skeleton such as 2,3,4,6-tetrafluoro-7,7,8,8-tetracyanoquinodimethane, and organic compounds each having a pyridine skeleton such as 4-tert-butylpyridine.

### [Electron-transporting Layer]

In the photoelectric conversion element of the present invention, the electron-transporting layer 4 may be arranged between the second electrode 3 and the photoelectric conversion layer 5 as illustrated in each of Fig. 1 and Fig. 2.

A material for the electron-transporting layer 4 is not particularly limited, and examples thereof include an N-type conductive polymer, an N-type low-molecular-weight organic semiconductor, an N-type metal oxide, an N-type metal sulfide, a halogenated alkali metal, an alkali metal, and a surfactant. Specific examples thereof include a cyano group-containing polyphenylene vinylene, a boron-containing polymer, bathocuproine, bathophenanthroline, hydroxyquinolinatoaluminum, an oxadiazole compound, a benzimidazole compound, a naphthalenetetracarboxylic acid compound, a fullerene compound, a perylene compound, a phosphine oxide compound, a phosphine sulfide compound, a fluoro group-containing phthalocyanine, titanium oxide, zinc oxide, indium oxide, tin oxide, gallium oxide, tin sulfide, indium sulfide, and zinc sulfide.

A preferred lower limit of the thickness of the electron-transporting layer 4 is 1 nm, and a preferred upper limit thereof is 2,000 nm. When such thickness is 1 nm or more, a hole can be sufficiently blocked, and when the thickness is 2,000 nm or less, the electron-transporting layer 4 is less liable to serve as a resistance at the time of the electron transportation, and hence the photoelectric conversion efficiency is improved. A more preferred lower limit of the thickness is 3 nm, a more preferred upper limit thereof is 1,000 nm, a still more preferred lower limit thereof is 5 nm, and a still more preferred upper limit thereof is 500 nm.

### <Application Examples>

Application examples of the present invention are directed to a photoelectric conversion apparatus, a moving body, and a building material.

### [Photoelectric Conversion Apparatus]

A photoelectric conversion apparatus may be formed by using the photoelectric conversion elements of the present invention. When the photoelectric conversion elements are connected, such photoelectric conversion apparatus may also be referred to as "photoelectric conversion cell" or "photoelectric conversion module." In the photoelectric conversion element, elements having different absorption wavelengths may be laminated to increase an output voltage. In addition, the photoelectric conversion apparatus includes the photoelectric conversion element of the present invention and an inverter. The inverter may be a converter for converting a DC voltage to an AC voltage. The photoelectric conversion apparatus may include an electricity storage unit connected to the photoelectric conversion element. The electricity storage unit is not limited as long as the electricity storage unit can store electricity. Examples thereof include a secondary battery using lithium ions, an all-solid-state battery, and an electric double layer capacitor. To impart a function of, for example, maintaining or increasing the amount of incident light, a surface layer to which water or dirt is hard to adhere, or a function of collecting or guiding light may be added.

### [Moving Body]

Fig. 3 is a perspective view for schematically illustrating a moving body including the photoelectric conversion element according to one embodiment of the present invention. A moving body 30 includes a photoelectric conversion element 31 of the present invention and a body 32 including the photoelectric conversion element 31. The photoelectric conversion element 31 is arranged on the position of the body 32 at which ambient light can be received. When the moving body 30 is an automobile, the photoelectric conversion element 31 may be arranged on a roof. Electric energy obtained by the photoelectric conversion element 31 may serve as the power of the moving body 30 or the power of any other electric equipment. Electric energy generated from the power of the moving body 30 may be used for the power of the photoelectric conversion element 31. When the moving body 30 is an automobile, friction energy generated with a brake may be converted into electric energy to be used for the control of the photoelectric conversion element 31.

The moving body 30 may be, for example, an automobile, a motorcycle, a railway vehicle, a ship, or a flying body including an artificial satellite, an airplane, and a drone. The configuration of the body 32 of the moving body 30 is not particularly limited, but is preferably formed of a material having high strength.

### [Building Material]

Fig. 4 is a perspective view for schematically illustrating a building material including the photoelectric conversion element according to one embodiment of the present invention. A building material 40 may be a roof of a building. The building material 40 of this embodiment includes a photoelectric conversion element 41 of the present invention, a protective member 42 for protecting the photoelectric conversion element 41, a heat dissipation member 43, and exteriors 44a and 44b.

The building material 40 of the present invention may include the heat dissipation member 43 having a thermal conductivity higher than that of the photoelectric conversion element 41. In general, when a building material including a photoelectric conversion element is used for a roof or the like, the temperature of the photoelectric conversion element 41 may be increased by sunlight, and hence the photoelectric conversion efficiency may be reduced. In this case, the reduction of the photoelectric conversion efficiency can be suppressed by using the heat dissipation member 43. Examples of the heat dissipation member 43 include a metal, an alloy, a liquid metal, and a liquid resin.

In addition, the building material 40 of the present invention may include the exteriors 44a and 44b. The exterior 44a and the exterior 44b may show different colors, or may show the same color. The exteriors 44a and 44b may be formed of the same member, or may be formed of different members. A paint or a transparent substrate may be used as an exterior member. An exterior member having small light absorption and a high heat-shielding property is preferably used.

In addition to the application examples described above, the following application examples are given: portable devices, such as a calculator, a sensor, and a small solar panel; wearable devices, such as a glasses-type terminal, a watch-type terminal, and a portable medical device; sheet structures supported by frames, such as a tent, a plastic greenhouse, and a loading platform of a truck; and structures to be used by being fixed, such as a road surface panel, a floating panel, a building material utilizing the flexibility of a substrate, a wall-type building material, a glass-type building material, and a mega solar panel.

### [Method of producing Photoelectric Conversion Element]

A method of producing the photoelectric conversion element of the present invention includes the steps of: forming a first electrode; forming a second electrode; and forming a photoelectric conversion layer containing a crystal having a perovskite structure between the first electrode and the second electrode.

The respective steps of the production method are described below.

### [Step of forming First Electrode and Step of forming Second Electrode]

The method of producing a photoelectric conversion element of the present invention includes the steps of: forming the first electrode; and forming the second electrode. In the step of forming the first electrode and the step of forming the second electrode, an appropriate method may be selected in accordance with a material for the first electrode and a material for the second electrode, respectively. Examples of such method include, but are not limited to, a sputtering method, a vacuum vapor deposition method, a vapor phase growth method (CVD method), and a spray pyrolysis deposition method (SPD method). The materials for the first electrode and the second electrode are as described above. When one, or each of both, of the first electrode and the second electrode is a transparent electrode, the thickness of the transparent electrode is preferably 0.03 to 3 µm.

When a solar cell is produced, cutting processing may be performed for circuit formation between steps. Examples of the cutting processing include mechanical patterning and laser patterning.

### [Modularization Step]

An element formed up to the electrode may be sealed. A sealing method is, for example, sealing with a resin or sealing with a film. Examples of a material used for the sealing include a silazane, a silicone rubber, a resin having a siloxane skeleton, and glass.

In addition, hairline treatment may be performed on the surface of the sealed element from the viewpoint of the suppression of adhesion between elements occurring during winding in a roll-to-roll system.

### [Step of forming Photoelectric Conversion Layer]

The step of forming the photoelectric conversion layer may include a step of applying a liquid containing the material for the photoelectric conversion layer described above. Examples of a method for the application include a spin coating method, a blade coating method, a slit die coating method, a screen printing method, a bar coater method, a casting method, a printing transfer method, a dip-up method, an ink jet method, a spray method, and a vacuum vapor deposition method. The method is appropriately selected therefrom in accordance with the properties of a photoelectric conversion layer to be produced, such as thickness control and orientation control.

Annealing treatment may be performed under reduced pressure or under an inert atmosphere (under a nitrogen or argon atmosphere) in order to remove a solvent or a dispersion medium from the applied liquid containing the material for the photoelectric conversion layer. The temperature of the annealing treatment is preferably 40 to 300°C, more preferably 50 to 150°C. The annealing treatment is preferably performed because materials for forming the respective layers may permeate each other at an interface between laminated layers to increase a contact area, and hence a short-circuit current can be increased.

### [Step of forming Charge-transporting Layer]

As the step of forming the charge-transporting layer, a method including applying a liquid containing the material for the charge-transporting layer described above is preferred. Examples of a method for the application include a spin coating method, a blade coating method, a slit die coating method, a screen printing method, a bar coater method, a casting method, a printing transfer method, a dip-up method, an ink jet method, a spray method, and a vacuum vapor deposition method. In addition, examples of the step of forming the charge-transporting layer include: a method including arranging a crystal of a cyclic conjugated compound in which pyrrole rings are bonded by conjugated bonds, and then applying a resin; a method including applying a resin solution in which a resin is dissolved, and then arranging a crystal of a cyclic conjugated compound in which pyrrole rings are bonded by conjugated bonds; and a method including applying a solution in which a crystal of a cyclic conjugated compound in which pyrrole rings are bonded by conjugated bonds is dispersed in a resin solution in which a resin is dissolved.

### <Method of identifying Amount of Compound>

The cyclic compound in which the pyrrole rings are bonded by covalent bonds in the charge-transporting layer to be used in the present invention and the organic compound in the crystal of the cyclic conjugated compound were each recognized by the following method.

The electrode surface of a photoelectric conversion element was peeled off so that the surface of the charge-transporting layer was exposed. The surface of the charge-transporting layer was wiped with a cotton swab or the like with a solvent such as chloroform so that the charge-transporting layer was exposed. The exposed charge-transporting layer was peeled off and collected. The kind of the organic compound of the present invention was identified by subjecting the collected charge-transporting layer to ¹H-NMR, MALDI-TOF-MS, IR, and gas chromatography. In addition, the pKa in the present invention was measured by subjecting the organic compound identified by the above-mentioned method to potentiometric titration.

In addition, the thickness of the layer was determined with a cross-sectional SEM (apparatus: SmartSEM manufactured by Carl Zeiss Co., Ltd.) after the cutting of the photoelectric conversion element and the fixing of the sample to a tilted sample stage. The crystallinity of the cyclic conjugated compound of the present invention was recognized by detecting diffraction peaks by XRD measurement (apparatus: X-ray diffractometer RINT-TTRII manufactured by Rigaku Corporation).

### [Examples]

The present invention is described in more detail below by way of Examples and Comparative Examples. The present invention is by no means limited to the following Examples without departing from the gist thereof. In the description of the following Examples, the term "part(s)" is by mass unless otherwise specified.

### <Production of Particle 1>

### Step (1)

Under a nitrogen flow atmosphere, 5.46 parts of orthophthalonitrile and 45 parts of α-chloronaphthalene were loaded into a reaction kettle. After that, the mixture was heated so that its temperature was increased to 30°C, followed by the maintenance of the temperature. Next, 3.75 parts of gallium trichloride was loaded into the mixture at the temperature (30°C). The moisture concentration of the mixed liquid at the time of the loading was 150 ppm. After that, the temperature of the mixed liquid was increased to 200°C. Next, under a nitrogen flow atmosphere, the mixed liquid was subjected to a reaction at a temperature of 200°C for 4 hours, and was then cooled. The product was filtered when its temperature reached 150°C. The resultant filter residue was subjected to dispersion washing with N,N-dimethylformamide at a temperature of 140°C for 2 hours, and was then filtered. The resultant filter residue was washed with methanol, and was then dried to provide a chlorogallium phthalocyanine particle in a yield of 65%.

### Step (2)

4.65 Parts of the chlorogallium phthalocyanine particle was dissolved in 139.5 parts of concentrated sulfuric acid at a temperature of 10°C, and the solution was dropped into 620 parts of ice water under stirring so that the particle was reprecipitated, followed by filtration with a filter press under reduced pressure. At this time, No. 5C (manufactured by Advantec Toyo Kaisha, Ltd.) was used as a filter. The resultant wet cake (filter residue) was subjected to dispersion washing with 2% ammonia water for 30 minutes, and was then filtered with the filter press. Next, the resultant wet cake (filter residue) was subjected to dispersion washing with ion-exchanged water, and then its filtration with the filter press was repeated three times. Finally, the filter residue was freeze-dried to provide a crude hydroxygallium phthalocyanine particle (hydrous hydroxygallium phthalocyanine particle) having a solid content of 23 mass% in a yield of 71%. The hydroxygallium phthalocyanine particle was dried with a hyper-dry dryer (product name: HD-06R, frequency (oscillatory frequency): 2,455 MHz±15 MHz, manufactured by Biocon (Japan) Ltd.). Thus, a hydroxygallium phthalocyanine (OHGaPc) particle (crystal) was obtained.

### Step (3)

2 Parts of the hydroxygallium phthalocyanine particle was mixed with 5 parts of an N-methylformamide solvent, and the mixture was subjected to dispersion treatment at 500 rpm for 20 hours with a sand mill (TSG-1, manufactured by Igarashi Machine Production Co., Ltd. (currently AIMEX Co., Ltd.), disc diameter: 70 mm, number of discs: 5) containing 5 parts of glass beads, followed by filtration and drying to provide a particle 1.

### <Production of Particles 2 to 18>

Particles 2 to 5, 14, 15, and 18 were each produced in the same manner as in the production of the particle 1 except that: in Step (3) in the production of the particle 1, such a solvent (organic compound) as shown in Table 2 below was used instead of N-methylformamide; and the treatment time and the number of revolutions in Step (3) in the production of the particle 1 were changed. Particles 10 to 12 and 17 were each produced in the same manner as in the production of the particle 1 except that: in Step (1) in the production of the particle 1, gallium trichloride was changed to each of various corresponding metal compounds, and the amount ratio between the particle and the solvent, the various reaction conditions, and the like were changed; and in Step (3) in the production of the particle 1, the solvent was changed to the solvent shown in Table 2. Particles 6 to 9 were each produced in the same manner as in the particle 1 except that, in Step (3) in the production of the particle 1, the amount ratio between the particle and the solvent, the treatment time, and the number of revolutions were changed.

A particle 13 was produced in the same manner as in the particle 1 except that: Step (2) in the production of the particle 1 was not performed; and the particle obtained in Step (1) was subjected to the treatment in Step (3) as it was. A particle 16 was produced in accordance with the method described in Japanese Patent No. 5132013.

### [Table 2]

**Table 2**

| Particle | (Solvent in Step 3) |
|---|---|
| Particle 1 | NMF |
| Particle 2 | IPA |
| Particle 3 | Phenol |
| Particle 4 | NEF |
| Particle 5 | NPF |
| Particle 6 | NMF |
| Particle 7 | NMF |
| Particle 8 | NMF |
| Particle 9 | NMF |
| Particle 10 | NMF |
| Particle 11 | NMF |
| Particle 12 | NMF |
| Particle 13 | NMF |
| Particle 14 | DMSO |
| Particle 15 | Ethylene glycol |
| Particle 16 | NMF |
| Particle 17 | Toluene |
| Particle 18 | DMF |

(In Table 2, NMF represents N-methylformamide, IPA represents 2-propanol, NEF represents N-ethylformamide, NPF represents N-propylformamide, DMSO represents dimethyl sulfoxide, and DMF represents N,N-dimethylformamide.)

### (Example 1)

### [Formation of Electron-transporting Layer]

A glass substrate with ITO was washed, and tin(II) oxide whose concentration had been adjusted to 3 mass% was applied thereonto by spin coating with a spin coater. After that, the resultant was heated at 150°C for 30 minutes to form an electron-transporting layer as a thin film having a thickness of 15 nm.

### [Formation of Photoelectric Conversion Layer]

17.9 Milligrams of methylammonium bromide, 137.6 mg of formamidinium iodide, and 460.8 mg of lead iodide were dissolved in 600 µL of N,N-dimethylformamide and 160 µL of dimethyl sulfoxide, and the solution was stirred for 1 hour (solution 1). Further, 389.72 mg of cesium iodide was dissolved in 1,000 µL of dimethyl sulfoxide, and the solution was stirred for 1 hour (solution 2). After that, 34 µL of the cesium iodide solution (solution 2) was added to the solution 1 to prepare a coating liquid for a photoelectric conversion layer. The coating liquid was applied onto the electron-transporting layer by spin coating to form a photoelectric conversion layer formed of Cs_{0.05}(FA_{0.83}MA_{0.17})_{0.96}Pb(I_{0.95}Br_{0.05})₃ and having a thickness of 400 nm.

### [Formation of Charge-transporting Layer]

1.0 Grams of polyvinyl butyral (product name: BM-2, manufactured by Sekisui Chemical Co., Ltd.) was dissolved in 19 g of 2-propanol by stirring for 24 hours to provide a resin solution 1.

0.1 Grams of the particle 1 and 0.01 g of a calixarene compound (Exemplified Compound 1 of Japanese Patent Laid-Open No. 2003-207913) were mixed with 12.8 g of 2-propanol, and 11 g of zirconia beads were loaded into the mixture, followed by dispersion with a paint shaker (manufactured by Toyo Seiki Seisaku-sho, Ltd.) for 6 hours. After that, 0.2 g of the resin solution 1 was added thereto, and dispersion with the paint shaker was performed again for 6 hours to prepare a coating liquid for a charge-transporting layer. The coating liquid for a charge-transporting layer was applied onto the photoelectric conversion layer by spin coating to form a charge-transporting layer having a thickness of 150 nm.

### [Introduction of Second Charge-transporting Layer]

0.098 Grams of Spiro-OMeTAD serving as a hole-transporting substance was dissolved in 2.2 g of chlorobenzene. 36 Microliters of an acetonitrile solution obtained by dissolving 0.13 g of lithium bis(trifluoromethanesulfonyl)imide in 0.30 g of acetonitrile and 24 µL of t-butylpyridine (TBP) were added to the chlorobenzene solution, and the contents were mixed. Further, 58 µL of an acetonitrile solution obtained by dissolving 0.10 g of [tris(2-(1H-pyrazol-1-yl)-4-tert-butylpyridine)cobalt(III) tris(bis(trifluoromethylsulfonyl)imide)] in 0.30 g of acetonitrile was mixed thereinto to prepare a coating liquid for a second charge-transporting layer. The coating liquid was applied onto the above-mentioned charge-transporting layer by a spin coating method to form a hole-transporting layer having a thickness of 130 nm.

### [Formation of First Electrode]

A gold electrode having a thickness of 80 nm and an area of 0.09 cm² was formed on the second charge-transporting layer by a vacuum vapor deposition method. Thus, a photoelectric conversion element was obtained.

### (Example 2)

In Example 2, a photoelectric conversion element was produced in the same manner as in Example 1 except that, in the formation of the charge-transporting layer, the particle 1 was changed to the particle 2.

### (Example 3)

In Example 3, a photoelectric conversion element was produced in the same manner as in Example 1 except that, in the formation of the charge-transporting layer, the particle 1 was changed to the particle 3.

### (Example 4)

In Example 4, a photoelectric conversion element was produced in the same manner as in Example 1 except that, in the formation of the charge-transporting layer, the particle 1 was changed to the particle 4.

### (Example 5)

In Example 5, a photoelectric conversion element was produced in the same manner as in Example 1 except that, in the formation of the charge-transporting layer, the particle 1 was changed to the particle 5.

### (Example 6)

In Example 6, a photoelectric conversion element was produced in the same manner as in Example 1 except that, in the formation of the charge-transporting layer, the particle 1 was changed to the particle 6.

### (Example 7)

In Example 7, a photoelectric conversion element was produced in the same manner as in Example 1 except that, in the formation of the charge-transporting layer, the particle 1 was changed to the particle 7.

### (Example 8)

In Example 8, a photoelectric conversion element was produced in the same manner as in Example 1 except that, in the formation of the charge-transporting layer, the particle 1 was changed to the particle 8.

### (Example 9)

In Example 9, a photoelectric conversion element was produced in the same manner as in Example 1 except that, in the formation of the charge-transporting layer, the particle 1 was changed to the particle 9.

### (Example 10)

In Example 10, a photoelectric conversion element was produced in the same manner as in Example 1 except that, in the formation of the charge-transporting layer, the particle 1 was changed to the particle 10, and polyvinyl butyral was not added.

### (Example 11)

In Example 11, a photoelectric conversion element was produced in the same manner as in Example 1 except that, in the formation of the charge-transporting layer, the particle 1 was changed to the particle 11, and polyvinyl butyral was not added.

### (Example 12)

In Example 12, a photoelectric conversion element was produced in the same manner as in Example 1 except that, in the formation of the charge-transporting layer, the particle 1 was changed to the particle 12, and polyvinyl butyral was not added.

### (Example 13)

In Example 13, a photoelectric conversion element was produced in the same manner as in Example 1 except that, in the formation of the charge-transporting layer, the particle 1 was changed to the particle 13, and polyvinyl butyral was not added.

### (Example 14)

In Example 14, a photoelectric conversion element was produced in the same manner as in Example 1 except that, in the formation of the charge-transporting layer, the particle 1 was changed to the particle 14.

### (Example 15)

In Example 15, a photoelectric conversion element was produced in the same manner as in Example 1 except that, in the formation of the charge-transporting layer, the particle 1 was changed to the particle 15.

### (Example 16)

In Example 16, a photoelectric conversion element was produced in the same manner as in Example 1 except that, in the formation of the charge-transporting layer, the particle 1 was changed to the particle 16.

### (Example 17)

In Example 17, a photoelectric conversion element was produced in the same manner as in Example 1 except that, in the formation of the charge-transporting layer, the addition amount of polyvinyl butyral was changed.

### (Example 18)

In Example 18, a photoelectric conversion element was produced in the same manner as in Example 1 except that, in the formation of the charge-transporting layer, polyvinyl butyral was not added.

### (Comparative Example 1)

In Comparative Example 1, a photoelectric conversion element was produced in the same manner as in Example 1 except that, in the formation of the charge-transporting layer, the particle 1 was changed to the particle 17, and polyvinyl butyral was not added.

### (Comparative Example 2)

In Comparative Example 1, a photoelectric conversion element was produced in the same manner as in Example 1 except that, in the formation of the charge-transporting layer, the particle 1 was changed to the particle 18, and polyvinyl butyral was not added.

### (Comparative Example 3)

In Comparative Example 3, a photoelectric conversion element was produced in the same manner as in Example 1 except that the charge-transporting layer was not formed.

### (Comparative Example 4)

In Comparative Example 4, a photoelectric conversion element was produced in the same manner as in Example 1 except that, in the formation of the charge-transporting layer, the particle 1 was changed to copper phthalocyanine (manufactured by TCI). When the copper phthalocyanine was analyzed, no organic compound in the crystal was detected.

Examples 1 to 18 and Comparative Examples 1 to 4 are shown in Table 3.

### [Table 3]

**Table 3**

| Example | Cyclic conjugated compound | Organic compound in crystal | | | | | P/B |
|---|---|---|---|---|---|---|---|
| | | Kind of organic compound | pKa | Dipole moment | Molar volume [cm³/mol] | Content in crystal [%] | |
| Example 1 | Hydroxygallium phthalocyanine | NMF | 16.5 | 3.90 | 67.6 | 1.46 | 10 |
| Example 2 | Hydroxygallium phthalocyanine | IPA | 16.5 | 1.66 | 67.6 | 1.46 | 10 |
| Example 3 | Hydroxygallium phthalocyanine | Phenol | 16.5 | 3.90 | 87.8 | 1.46 | 10 |
| Example 4 | Hydroxygallium phthalocyanine | NEF | 16.7 | 3.90 | 84.1 | 1.21 | 10 |
| Example 5 | Hydroxygallium phthalocyanine | NPF | 16.8 | 3.90 | 100.6 | 1.05 | 10 |
| Example 6 | Hydroxygallium phthalocyanine | NMF | 16.5 | 3.90 | 67.6 | 1.98 | 10 |
| Example 7 | Hydroxygallium phthalocyanine | NMF | 16.5 | 3.90 | 67.6 | 2.20 | 10 |
| Example 8 | Hydroxygallium phthalocyanine | NMF | 16.5 | 3.90 | 67.6 | 0.52 | 10 |
| Example 9 | Hydroxygallium phthalocyanine | NMF | 16.5 | 3.90 | 67.6 | 0.30 | 10 |
| Example 10 | Copper phthalocyanine | NMF | 16.5 | 3.90 | 67.6 | 1.50 | - |
| Example 11 | Titanyl phthalocyanine | NMF | 16.5 | 3.90 | 67.6 | 1.50 | - |
| Example 12 | Zinc phthalocyanine | NMF | 16.5 | 3.90 | 67.6 | 1.50 | - |
| Example 13 | Chlorogallium phthalocyanine | NMF | 16.5 | 3.90 | 67.6 | 1.30 | - |
| Example 14 | Hydroxygallium phthalocyanine | DMSO | 41.0 | 4.00 | 71.0 | 1.30 | 10 |
| Example 15 | Hydroxygallium phthalocyanine | Ethylene glycol | 16.5 | 2.30 | 56.5 | 1.30 | 10 |
| Example 16 | 2,3,7,8,12,13,17,18-Octaphenyl-5,10,15,20-tetraazaporphyrin | NMF | 16.5 | 3.90 | 67.6 | 1.46 | 10 |
| Example 17 | Hydroxygallium phthalocyanine | NMF | 16.5 | 3.90 | 67.6 | 1.46 | 20 |
| Example 18 | Hydroxygallium phthalocyanine | NMF | 16.5 | 3.90 | 67.6 | 1.46 | - |
| Comparative Example 1 | Titanyl phthalocyanine | Toluene | 16.5 | 0.36 | 105.7 | 1.30 | - |
| Comparative Example 2 | Hydroxygallium phthalocyanine | DMF | 0.44 | 3.80 | 82.6 | 1.35 | - |
| Comparative Example 3 | - | - | - | - | - | - | - |
| Comparative Example 4 | Copper phthalocyanine | - | - | - | - | - | - |

| | | | | | | | |
|---|---|---|---|---|---|---|---|
| (In Table 3, abbreviations such as NMF represent the same compounds as those in Table 2.) | | | | | | | |

### [Evaluation]

The photoelectric conversion element obtained in each of Examples and Comparative Examples was subjected to the following evaluation.

### (Power Generation Efficiency Evaluation)

A power supply (236 model, manufactured by Keithley Instruments) was connected between the electrodes of the photoelectric conversion element of Example 1, and its photoelectric conversion efficiency was evaluated by: irradiating the element with constant light through use of a solar simulator (manufactured by Yamashita Denso Corporation) having an intensity of 100 mW/cm²; and measuring the generated current and voltage. In addition, the produced photoelectric conversion element was left to stand for 1 month under an environment at a temperature of 30°C and a humidity of 60%RH, and then its photoelectric conversion efficiency was determined by the same measurement method as that described above. A value of the photoelectric conversion efficiency after the standing for 1 month with respect to the initial photoelectric conversion efficiency was defined as a maintenance rate of the photoelectric conversion efficiency. The photoelectric conversion elements of Examples 1 to 18 and Comparative Examples 1 to 3 were each also evaluated in the same manner as in Example 1, and its photoelectric conversion efficiency and its maintenance rate of the photoelectric conversion efficiency were determined. The results are shown in Table 4.

### [Table 4]

**Table 4**

| Example | Photoelectric conversion efficiency [%] | Maintenance rate of photoelectric conversion efficiency [%] |
|---|---|---|
| Example 1 | 18.5 | 95 |
| Example 2 | 12.4 | 94 |
| Example 3 | 12.6 | 93 |
| Example 4 | 17.3 | 95 |
| Example 5 | 14.1 | 93 |
| Example 6 | 17.1 | 92 |
| Example 7 | 16.2 | 87 |
| Example 8 | 16.1 | 94 |
| Example 9 | 13.1 | 96 |
| Example 10 | 13.5 | 81 |
| Example 11 | 15.1 | 78 |
| Example 12 | 14.5 | 81 |
| Example 13 | 15.9 | 91 |
| Example 14 | 17.8 | 92 |
| Example 15 | 13.5 | 90 |
| Example 16 | 15.9 | 85 |
| Example 17 | 15.2 | 84 |
| Example 18 | 16.1 | 81 |
| Comparative Example 1 | 11.4 | 93 |
| Comparative Example 2 | 14.8 | 85 |
| Comparative Example 3 | 11.5 | 75 |
| Comparative Example 4 | 11.6 | 79 |

The present invention is not limited to the embodiments described above, and various changes and modifications may be made without departing from the spirit and scope of the present invention. The following claims are appended hereto in order to make the scope of the present invention public.

The present application claims priority based on Japanese Patent Application No. 2023-184761 filed on October 27, 2023, Japanese Patent Application No. 2023-184756 filed on October 27, 2023, Japanese Patent Application No. 2023-184750 filed on October 27, 2023, Japanese Patent Application No. 2023-216294 filed on December 21, 2023, Japanese Patent Application No. 2023-216296 filed on December 21, 2023, Japanese Patent Application No. 2023-216299 filed on December 21, 2023, Japanese Patent Application No. 2024-022244 filed on February 16, 2024, Japanese Patent Application No. 2024-022251 filed on February 16, 2024, Japanese Patent Application No. 2024-022246 filed on February 16, 2024, Japanese Patent Application No. 2024-086004 filed on May 28, 2024, and Japanese Patent Application No. 2024-186452 filed on October 23, 2024, and the entire contents thereof are incorporated herein by reference.

### [Reference Signs List]

1 photoelectric conversion element
2 substrate
3 second electrode
4 electron-transporting layer
5 photoelectric conversion layer
6 charge-transporting layer
7 first electrode
30 moving body
31, 41 photoelectric conversion element
32 body
40 building material
42 protective member
43 heat dissipation member
44a, 44b exterior

## Claims

1. A photoelectric conversion element comprising:
a first electrode;
a second electrode; and
a photoelectric conversion layer arranged between the first electrode and the second electrode, the photoelectric conversion layer containing a crystal having a perovskite structure,
wherein the photoelectric conversion element comprises, between the photoelectric conversion layer and the first electrode, a layer containing a crystal of a cyclic conjugated compound in which pyrrole rings are bonded by conjugated bonds,
wherein the crystal of the cyclic conjugated compound in which the pyrrole rings are bonded by conjugated bonds contains an organic compound, and
wherein the organic compound has a pKa of 10.0 or more and a dipole moment of 1.60 D or more.

2. The photoelectric conversion element according to claim 1, wherein the organic compound has a molar volume of 20.0 to 85.0 cm³/mol.

3. The photoelectric conversion element according to claim 1 or 2, wherein a content of the organic compound is 0.50 to 2.00 mass% with respect to the cyclic conjugated compound in which the pyrrole rings are bonded by conjugated bonds.

4. The photoelectric conversion element according to any one of claims 1 to 3, wherein the cyclic conjugated compound in which the pyrrole rings are bonded by conjugated bonds contains any one of Ga, Ti, or Zn as a central element.

5. The photoelectric conversion element according to claim 4, wherein the central element is Ga.

6. The photoelectric conversion element according to any one of claims 1 to 5, wherein the cyclic conjugated compound in which the pyrrole rings are bonded by conjugated bonds has at least one selected from the group consisting of: OH; Cl; and O as an axial ligand.

7. The photoelectric conversion element according to any one of claims 1 to 6, wherein the pKa of the organic compound is 10.0 to 40.0.

8. The photoelectric conversion element according to any one of claims 1 to 7, wherein the dipole moment of the organic compound is 2.20 D or more.

9. The photoelectric conversion element according to any one of claims 1 to 8, wherein the organic compound is any one selected from the group consisting of: N-methylformamide; N-ethylformamide; N-propylformamide; and dimethyl sulfoxide.

10. The photoelectric conversion element according to any one of claims 1 to 9,
wherein the layer containing the crystal of the cyclic conjugated compound in which the pyrrole rings are bonded by conjugated bonds contains a resin, and
wherein, when a content of the resin in the layer is represented by B and a content of the cyclic conjugated compound in which the pyrrole rings are bonded by conjugated bonds therein is represented by P, a ratio P/B is 1 to 20.

11. The photoelectric conversion element according to any one of claims 1 to 10, wherein the cyclic conjugated compound in which the pyrrole rings are bonded by conjugated bonds is a phthalocyanine compound.

12. A photoelectric conversion apparatus comprising the photoelectric conversion element of any one of claims 1 to 11.
